(19) Europäisches Patentamt
European Patent Office
Office européen des brevets

(11) Veröffentlichungsnummer: **0 409 769 A1**

# (12) EUROPÄISCHE PATENTANMELDUNG

(21) Anmeldenummer: **90810414.4**

(22) Anmeldetag: **07.06.90**

(51) Int. Cl.⁵: **A61F 2/30, A61B 17/58**

(30) Priorität: **18.07.89 CH 2682/89**

(43) Veröffentlichungstag der Anmeldung:
**23.01.91 Patentblatt 91/04**

(84) Benannte Vertragsstaaten:
**AT DE FR GB IT**

(71) Anmelder: **GEBRÜDER SULZER
AKTIENGESELLSCHAFT
Zürcherstrasse 9
CH-8401 Winterthur(CH)**

(72) Erfinder: **Willert, Hans Georg, Prof., Dr., med.
Schlegelweg 9
D-3400 Göttingen(DE)**
Erfinder: **Bürgi, Maya
Chaennerwisstrasse 5
CH-8352 Räterschen(CH)**
Erfinder: **Koch, Rudolf
Oberdorfstrasse 229
CH-8267 Berlingen(CH)**

(54) **Armierung eines Knochenzementbettes.**

(57) Die Erfindung handelt von einer Armierung (4) für den proximalen Bereich eines Knochenzementbettes (3) für eine Femurkopfprothese (1), wobei diese aus einem Gitter von sich kreuzenden Stegen (5, 6) besteht, von denen mindestens ein Teil wellenförmig ausgebildet ist. Dadurch, dass Stege (5) des Gitters in Längsrichtung (7) eine überhöhte Wellenform aufweisen und bei Verkleinerung des Spaltes zwischen Knochenaushöhlung (14) und Prothesenschaft (1) einfedern, wird die Prothese unter Ausmittelung der Rückstellkräfte und der Spaltweite mechanisch fixiert bis der Knochenzement ausgehärtet ist.

Fig.1

# ARMIERUNG EINES KNOCHENZEMENTBETTES

Die Erfindung betrifft die Armierung für den proximalen Bereich eines Knochenzementbettes für eine Femurkopfprothese, wobei diese Armierung aus einem Gitter von sich kreuzenden Stegen besteht, von denen mindestens ein Teil wellenförmig ausgebildet ist.

Eine derartige Armierung ist beispielsweise aus der FR-PS 77 39 062 bekannt, wobei die Armierung strumpfartig über den Schaft gezogen wird, um anschliessend bei der Implantation zusammen mit dem Schaft in den zuvor in den Knochen eingebrachten Zement eingepresst zu werden. Die Armierung stellt für später eine Verstärkung dar und bildet eine gewebeähnliche Textur, die entgegen der Einführrichtung der Prothese vom noch fliessfähigen Knochenzement umf los sen wird. Als nachteilig wirkt sich dabei das über die ganze Länge wiederholte schneiden des aufsteigenden Knochenzementes für eine spätere Rissbildung in den zusammenfliessenden Flächen aus. Ausserdem besteht allgemein eine Unsicherheit über die Lage der Prothese im Knochenzementbett und über die Zementverteilung, bis die Verfestigung und das Aushärten stattgefunden haben.

Hier schafft die Erfindung Abhilfe. Sie löst die Aufgabe, die Lage der Prothese mit dem Einbringen mechanisch festzulegen und die Erhitzung des Knochenzementes bei Aushärung mit Wärmeentwicklung niedrig zu halten. Gemäss der Erfindung wird die Aufgabe gelöst, indem die Stege des Gitters eine Wellenform aufweisen, deren Scheitelhöhe einem Mehrfachen der Dicke der Stege entspricht, indem die Stege mit wellenform in Längsrichtung zwischen den Kreuzungspunkten mindestens zwei Halbwellen bilden und indem die Stege in den Kreuzungspunkten fest miteinander verbunden sind.

Die Vorteile der Erfindung sind darin zu sehen, dass vor dem Aushärten des Knochenzementes die Lage der Prothese vorfixiert ist, dass in dieser Lage zwangsläufig durch ein Ausmitteln der Rückstellkräfte zur Prothese die Spaltweite ausgemittelt ist und dass diese Lage kontrollierbar und bei weiterem Eindrücken der Prothese veränderbar ist.

Im folgenden wird die Erfindung anhand von Ausführungsbeispielen beschrieben. Es zeigen:

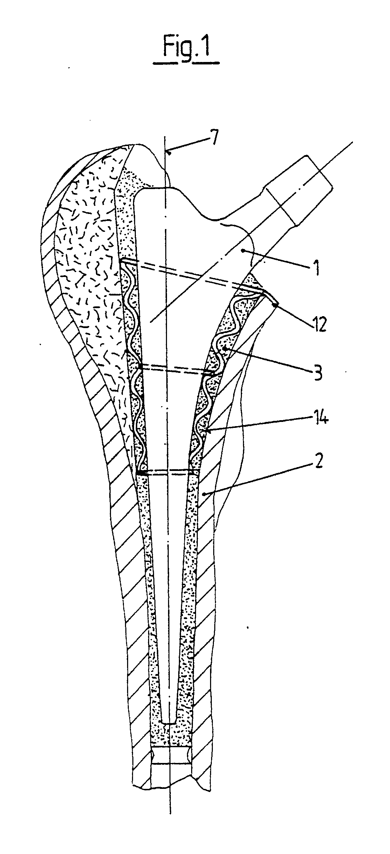

Fig. 1 schematisch die Seitenansicht eines Vertikalschnittes durch den proximalen Teil eines Femurknochens mit implantiertem Schaft und mit Armierungsgitter für eine Femurkopfprothese;

Fig. 2 die Seitenansicht eines Armierungsgitters;

Fig. 3 die Seitenansicht eines Vertikalschnittes durch ein Armierungsgitter entsprechend Fig. 2;

Fig. 4 die Seitenansicht eines Vertikalschnittes durch ein Armierungsgitter mit rhombisch verlaufenden Stegen;

Fig. 5 schematisch die Seitenansicht eines Vertikalschnittes durch den proximalen Teil eines Femurknochens mit implantiertem Prothesenschaft und mit einem Armierungsgitter mit unterbrochenen gewellten Stegen;

Fig. 6 einen vergrösserten perspektivischen Ausschnitt eines in die Knochenhöhle eingelegten Armierungsgitters mit unterbrochenen gewellten Stegen;

Fig. 7 schematisch die Seitenansicht eines Vertikalschnittes durch den proximalen Teil eines Femurknochens mit implantiertem Prothesenschaft und mit einem Armierungsgitter mit gewellten symmetrischen Doppelstegen;

Fig. 8 einen vergrösserten perspektivischen Ausschnitt eines in die Knochenhöhle eingelegten Armierungsgitters mit gewellten symmetrischen Doppelstegen und

Fig. 9 einen vergrösserten Querschnitt durch einen gewellten symmetrischen Doppelsteg und dessen Deformation bei Spaltverkleinerung.

In den Figuren sind Armierungen 4 für den proximalen Bereich eines Knochenzementbettes 3 für eine Femurkopfprothese 1 gezeigt, die aus einem Gitter von sich kreuzenden Stegen 5, 6, von denen mindestens ein Teil wellenförmig ausgebildet ist, bestehen. Erfindungsgemäss weisen Stege 5 des Gitters eine Wellenform auf, deren Scheitelhöhe einem Mehrfachen der Stegdicke entspricht. Im weiteren bilden die Stege 5 mit Wellenform in Längsrichtung zwischen den Kreuzungspunkten 8 mindestens zwei Halbwellen und sind diese Stege 5 in Kreuzungspunkten 8 fest miteinander verbunden.

Die verformten Gitter 4 entsprechen mit ihrer äusseren Umhüllenden der sich verjüngenden Aushöhlung 14 im proximalen Bereich des Femurknochens 2. Sie werden vor dem Einbringen des Knochenzementes in diese Aushöhlung 14 eingelegt, wobei sie mit Abstützungen 9 am Knochenrand 12 aufliegen. Mit dem Einführen des Prothesenschaftes 1 liegen die gewellten Stege 5 in den engsten Zwischenräumen zwischen Schaft 1 und Aushöhlung 14 auf und bilden einen Widerstand. Mit dem weiteren Eindrücken des Schaftes platten sich die als erste beidseitig abstützenden wellenförmigen Stege ab, bis entsprechend der Spaltverkleinerung praktisch alle dafür vorgesehenen Wellenabschnitte tragen. Gleichzeitig findet die Prothese 1 ihre Lage entsprechend dem Kräfteausgleich zwischen den Rückstellkräften der tragenden Wellenabschnitte, wobei die in Längsrichtung 7 eingebrachte Prothese durch die Selbsthemmung der Reibungskräfte in Längsrichtung und gegen Drehung fixiert ist und nach erfolgter Lagekontrolle durch weiteres Nachdrücken in ihrer Lage verändert werden kann. Entsprechend dem Kräfteausgleich am Prothesenschaft 1 wurde der Spalt zwischen Schaft und Knochenaushöhlung 14 durch die Stege in Wellenform ausgemittelt. Es entstehen somit kaum grössere Ansammlungen von Knochenzement im Spalt und beim Aushärten weniger Wärmestaus, die die Temperatur an der Knochenaushöhlung höher ansteigen lassen.

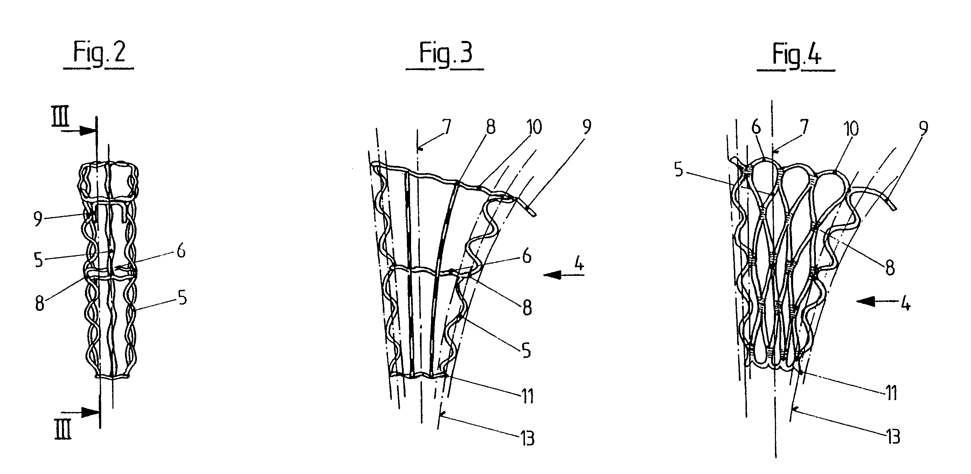

In Fig. 2 und 3 ist ein Armierungsgitter 4 mit durchgehenden gewellten Längsstegen 5 dargestellt. Beim Einbringen der Prothese erfolgt neben der Abplattung der gewellten Stege auch eine Längung in Längsrichtung. Die Querstege 6 sowie der obere Rand 10 und der untere Rand 11 liegen nahe bei der Mittellinie der Wellenform in Längsrichtung und weisen selber eine geringere Wellenhöhe auf, damit sie beim Einschieben des Armierungsgitters 4 nicht an der Aushöhlung 14 anstehen und damit genügend Spiel für ein Aufweiten durch den konischen Prothesenschaft 1 vorhanden ist.

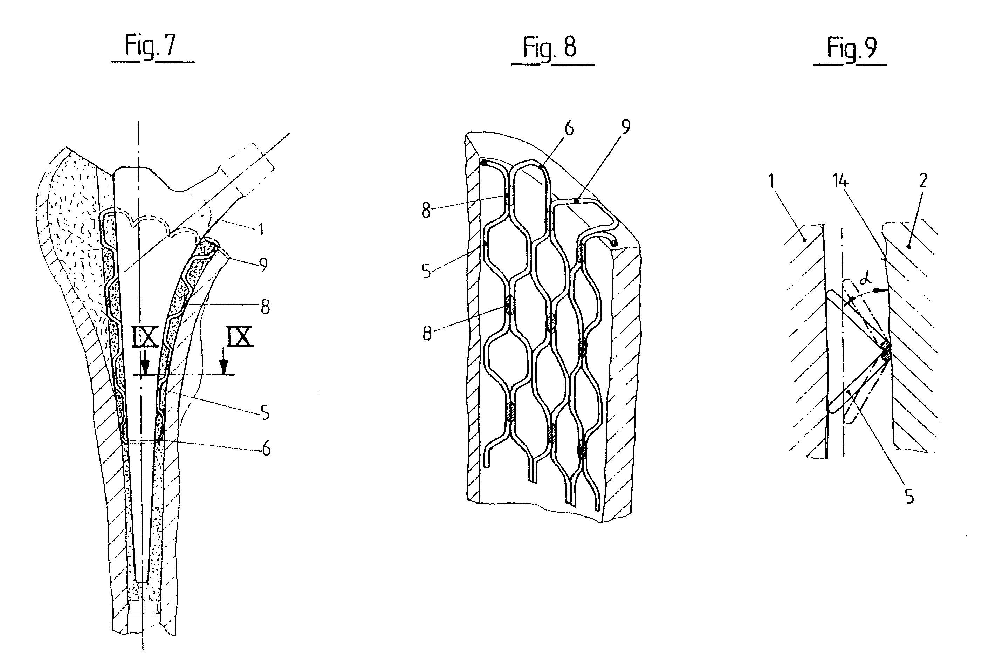

Fig. 4 zeigt eine Ausführung mit längsgewellten rhombischen Stegen 5. Ein Vorteil dieser Anordnung besteht darin, dass sich das Gitter 4 in Querrichtung in einem grossen Bereich aufweiten lässt und somit ein Gitter für verschiedene Schaftgrössen und -formen verwendbar ist. Aehnliche Vorteile bietet das Gitter in Fig. 7, 8, 9, das aus gewellten symmetrischen Doppelstegen 5 besteht, die im nicht deformierten Zustand unter einem Winkel α von annähernd 50° von der Oberfläche der Aushöhlung 14 wegstehen. Bei Belastung federn die Stege 5 seitlich weg und verkleinern den Winkel α ohne dass eine Längung des Gitters eintritt und ohne Veränderung der Abstützpunkte in der Aushöhlung 14.

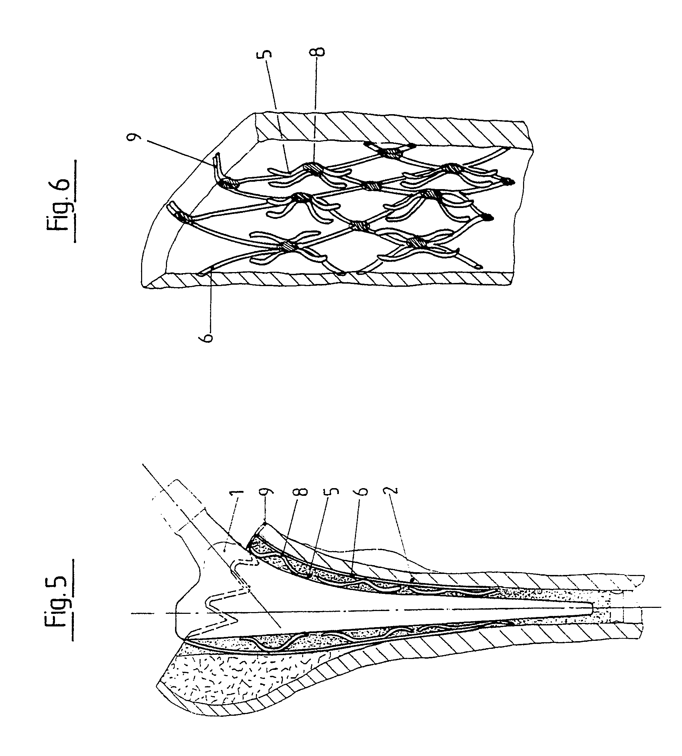

Fig. 5 und 6 zeigen ein flaches rhombisches Gitter mit guter Anpassungsfähigkeit an verschiedene Schaftformen 1, in das unterbrochene Stege 5 eingebaut sind, die sich über Kreuzungspunkte 8 starr verbunden wie am Rücken aufgehängte Vierfüssler selbst an der Schaftoberfläche 1 ausrichten.

## Ansprüche

1. Armierung (4) für den proximalen Bereich eines Knochenzementbettes (3) für eine Femurkopfprothese (1) bestehend aus einem Gitter von sich kreuzenden Stegen (5, 6), von denen mindestens ein Teil wellenförmig ausgebildet ist, dadurch gekennzeichnet, dass bei Stegen (5) des Gitters, die eine Wellenform aufweisen, die Scheitelhöhe einem Mehrfachen der Dicke entspricht, dass die Stege (5) mit Wellenform in Längsrichtung zwischen den Kreuzungspunkten (8) mindestens zwei Halbwellen bilden und dass die Stege (5, 6) in Kreuzungspunkten (8) fest miteinander verbunden sind.

2. Armierung nach Anspruch 1, dadurch gekennzeichnet, dass proximal am Rand (10) der Armierung Abstützungen (9) nach aussen von der Umhüllenden der Armierung wegstehen.

3. Armierung nach Anspruch 1 und 2, dadurch gekennzeichnet, dass distal der Rand (11) der Armierung annähernd auf der Mittellinie (13) der Wellenform liegt.

4. Armierung nach Anspruch 1 und 2, dadurch gekennzeichnet, dass quer zu den Mantellinien verlaufenden Stege (6) eine wesentlich geringere Wellenhöhe aufweisen.

5. Armierung nach einem der Ansprüche 1 oder 4, dadurch gekennzeichnet, dass sich die Stege (5, 6) beim Einbringen der Femurkopfprothese (1) durch Deformation den lokalen Verengungen der Spaltweite zwischen Prothese und Knochen anpassen und dass ihre Rückstellkräfte mit zunehmender Deformation wachsen.

6. Armierung nach einem der Ansprüche 1 bis 5, dadurch gekennzeichnet, dass die Wellenform der Stege (5) sinusähnlich ist.

7. Armierung nach einem der Ansprüche 1 bis 6, dadurch gekennzeichnet, dass gewellte Stege (5) zwischen den Knotenpunkten (8) Unterbrechungen aufweisen.

## Fig.1

Fig. 4

Fig. 3

Fig. 2

Fig. 6

Fig. 5

## Fig. 7

## Fig. 8

## Fig. 9

EP 0 409 769 A1

**Europäisches Patentamt**

# EUROPÄISCHER RECHERCHENBERICHT

Nummer der Anmeldung

**EP 90 81 0414**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y,D | FR-A-2 412 304 (THE SAMPSON CORP.)(20-07-1979)<br>* Seite 5, Zeile 26 - Seite 6, Zeile 7; Seite 6, Zeile 36 - Seite 7, Zeile 8; Figur 1 *<br>— — — | 1,2 | A 61 F 2/30<br>A 61 B 17/58 |
| Y | US-A-4 718 909 (BROWN)(12-01-1988)<br>* Spalte 5, Zeilen 37-50; Figur 8 *<br>— — — | 1,2 | |
| A | EP-A-0 224 890 (T.H. KARL-MARX-STADT)(10-06-1987)<br>* Seite 2, Zeilen 22-38; Figur 1C *<br>— — — | 1 | |
| A | GB-A-1 525 667 (BRANEMARK)(20-09-1978)<br>* Seite 3, Zeilen 3-6; Ansprüche 1,3; Figur 5 *<br>— — — | 1 | |
| A | FR-A-2 610 824 (CUILLERON)(19-08-1988)<br>* Seite 3, Zeilen 10-13; Figur 5 *<br>— — — — — | 1 | |

**RECHERCHIERTE SACHGEBIETE (Int. Cl.5)**

A 61 F
A 61 B
A 61 C

Der vorliegende Recherchenbericht wurde für alle Patentansprüche erstellt

| Recherchenort | Abschlußdatum der Recherche | Prüfer |
|---|---|---|
| Den Haag | 22 Oktober 90 | MOERS R.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument